# EUROPÄISCHE PATENTSCHRIFT

(11) **EP 2 809 761 B1**
(45) Veröffentlichungstag und Bekanntmachung des Hinweises auf die Patenterteilung: **08.02.2017**
(21) Anmeldenummer: 12809738.3
(22) Anmeldetag: 17.12.2012
(51) Int. Cl.: C11D 3/00, C11D 3/16, C11D 3/50, A61K 8/49, A61K 8/58, A61Q 13/00

(54) **KOMBINATIONEN AUS 1-AZA-3,7-DIOXABICYCLO[3.3.0]OCTAN-VERBINDUNGEN UND KIESELSÄUREESTERN UND IHRE VERWENDUNG ALS DUFTSTOFFVORLÄUFER**
COMBINATION OF 1-AZA-3,7-DIOXABICYCLO[3.3.0]OCTANE COMPOUNDS AND SILICIC ACID ESTER AND USE AS FRAGRANCE PRECURSOR
COMBINAISON DE 1-AZA-3,7-DIOXABICYCLO[3.3.0]OCTAN ET ESTER DE SILICE ET UTILISATION COMME PRECURSEUR DE PARFUME

(30) Priorität: 01.02.2012 DE 102012201424
(43) Veröffentlichungstag der Anmeldung: 10.12.2014
(73) Patentinhaber: Henkel AG & Co. KGaA, 40589 Düsseldorf (DE)
(72) Erfinder: BAUER, Andreas, 41564 Kaarst (DE); HUCHEL, Ursula, 50733 Köln (DE); GERIGK, Andreas, 41812 Erkelenz (DE); WEYHE, Marc, 47802 Krefeld (DE); GERKE, Thomas, 40627 Düsseldorf (DE); SMYREK, Hubert, 47804 Krefeld (DE)
(86) Internationale Anmeldenummer: PCT/EP2012/075717
(87) Internationale Veröffentlichungsnummer: WO 2013/113443

(56) Entgegenhaltungen:
- WO-A1-00/14091
- DE-A1-102006 003 092
- DE-A1-102006 060 943

## Beschreibung

Die Erfindung betrifft Kombinationen aus 1-Aza-3,7-dioxabicyclo[3.3.0]octan-Verbindungen mit Kieselsäureestern, ihre Verwendung als Duftstoffvorläufer und diese enthaltende Wasch- und Reinigungsmittel, Weichspüler und Kosmetika, sowie Verfahren zur Verlängerung des Duftempfindens von derartigen Mitteln und zur Bekämpfung von Schlechtgerüchen.

Neben dem direkten Zusatz von Duftstoffen zu Wasch- und Reinigungsmitteln, Weichspülern und Kosmetika wurde auch der Zusatz so genannter Duftstoffvorläufer vorgeschlagen. Duftstoffvorläufer stellen in Analogie zu den Pro-Drugs ein chemisches Derivat eines Duftstoffs dar, das beispielsweise die Flüchtigkeit des Duftstoffs vermindert und unter Umgebungsbedingungen eine zeitlich verzögerte Freisetzung des Duftstoffs erlaubt. Durch eine Derivatisierung von Duftstoffen wie Duftstoff-Aldehyden, Duftstoff-Ketonen oder Duftstoff-Alkoholen kann der Dampfdruck dieser Verbindungen erniedrigt werden. Da die Derivatisierungsreaktion reversibel ist, kann der chemisch gebundene Duftstoff-Aldehyd, das Duftstoff-Keton oder der Duftstoff-Alkohol unter bestimmten Bedingungen, beispielsweise Umgebungsbedingungen, an der Bindungsstelle gespalten werden. Dadurch wird der Duftstoff-Aldehyd oder Duftstoff-Keton wieder freigesetzt, was zu einem verlängerten Dufteindruck führen kann.
Zudem spielt neben der Verlängerung des Dufteindrucks die Bekämpfung von Schlechtgerüchen eine große Rolle.

In der US 6,861,402 sind Duftstoffvorläufer beschrieben, die einen Duftstoff-Aldehyd oder ein Duftstoff-Keton in Form eines Oxazolidins gebunden enthalten. Dabei wird beispielsweise N-Benzolethanolamin mit einem Duftstoff umgesetzt, so dass sich ein monocyclisches Oxazolidin ergibt.

WO 2004/009564 A2 betrifft cyclische Co-Tenside, die durch Kondensationsreaktion von C₃- bis C₆-Aldehyden mit mehrwertigen Alkoholen, Aminen, Thiolen oder Carbonsäuren entstehen. Die Co-Tenside eignen sich für den Einsatz in Haushaltswaschmitteln, Haushaltsreinigern, Körperreinigungsmitteln und Körperpflegemitteln.

WO 2000/14091 A1 betrifft oligomere Kieselsäureester, die Reste von Duftstoffalkoholen enthalten und sich beispielsweise zur Beduftung von Wasch- und Reinigungsmitteln eigenen, da sie bei der Hydrolyse duftende Alkohole freisetzen.

DE 10 2006 003 092 A1 betrifft 1-Aza-3,7-dioxabicyclo[3.3.0]octan-Verbindungen, Verfahren zu ihrer Herstellung, ihre Verwendung als Pro-Fragrances und diese enthaltende Wasch- und Reinigungsmittel, Weichspüler und Kosmetika, sowie ein Verfahren zur Verlängerung des Duftempfindens von derartigen Mitteln.

DE 10 2006 060 943 A1 betrifft Kieselsäureester, an denen Duftstoff, vorzugsweise als 1-Aza-3,7-dioxabicyclo[3.3.0]octan-Verbindungen oder als monocyclische Oxazolidine, gebunden sind, und sich beispielsweise zur Beduftung von Wasch- und Reinigungsmitteln eignen, da sie bei der Hydrolyse die gebundenen Duftstoff wieder freisetzen.

Aufgabe der vorliegenden Erfindung ist es nun, verbesserte Duftstoff-Vorformen, so genannte Duftstoffvorläufer bereitzustellen, die Schlechtgerüche, auch über einen Zeitraum von mehreren Tagen, nachhaltig überdecken können.

Der Begriff Schlechtgeruch ist dem Fachmann allgemein bekannt. Unter Schlechtgeruch im Sinne dieser Anmeldung werden alle Gerüche einbezogen, die in einer Gruppe von zehn Personen von mindestens 7 dieser Personen als unangenehm/schlecht eingestuft werden. Beispiele solcher Schlechtgerüche sind Schweißgeruch, Fäkalgeruch, Modergeruch, Bakteriell erzeugter Schlechtgeruch, Fischgeruch, der Geruch von C₁- bis C₁₅-Fettsäuren oder der Geruch von stundenlang stehender, nasser Wäsche.

Aufgabe der vorliegenden Erfindung ist weiterhin die Bereitstellung von Duftstoffvorläufern, die einen verlängerten Dufteindruck bei Duftstoff-Aldehyden, Duftstoff-Ketonen und Duftstoff-Alkoholen erlauben, die an sich einen hohen Dampfdruck aufweisen. Weiterhin sollen die mit den erfindungsgemäßen Verbindungen behandelten Substraten, einen angenehmen und langanhaltenden Duft erhalten.

Insbesondere bestand die Aufgabe darin, hydrolysestabile Duftstoffvorläufer bereitzustellen, die sich in wässrige Zusammensetzungen, beispielsweise in wässrige Wasch- und Reinigungsmittel einarbeiten lassen, und auch mehrere Tage nach der Anwendung und in Anwesenheit von Schlechtgerüchen, beispielsweise von nasser, gewaschener Wäsche, die in der Waschmaschine oder im Wäschekorb über mehrere Tage liegen gelassen wird, beim Anwender zu einem positiven Dufteindruck führen.

Überraschenderweise wurde nun gefunden, dass Kombinationen aus Duftstoffaldehyden und/oder Duftstoffketonen die als 1-Aza-3,7-dioxabicyclo[3.3.0]octan-Verbindungen (bicyclische Oxazolidin-Derivate) derivatisiert vorliegen mit Duftstoff-Alkoholen, die als Kieselsäureester derivatisiert vorliegen synergistische Eigenschaften bei der Bekämpfung und Überdeckung von Schlechtgerüchen, insbesondere über einen Zeitraum von mehreren Tagen aufweisen.

Die Aufgabe wird daher gelöst durch eine Kombination aus
(a) einer 1-Aza-3,7-dioxabicyclo[3.3.0]octan-Verbindung der allgemeinen Formel (I) 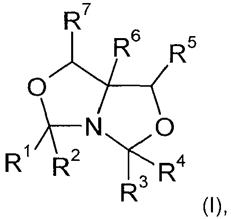 wobei
   R¹, R², R³, R⁴ unabhängig voneinander für Reste stehen, die in einer Verbindung der allgemeinen Formel R¹-C(=O)-R² oder R³-C(=O)-R⁴ einen Duftstoff-Aldehyd mit mindestens 6 Kohlenstoffatomen oder ein Duftstoff-Keton mit mindestens 6 Kohlenstoffatomen ergeben,
   R⁵, R⁶, R⁷ unabhängig voneinander für H oder einen Kohlenwasserstoff-Rest stehen, der acyclisch oder cyclisch, substituiert oder unsubstituiert, verzweigt oder unverzweigt sowie gesättigt oder ungesättigt sein kann
   oder Gemische dieser Verbindungen und
(b) einem Kieselsäureester der allgemeinen Formel (II) 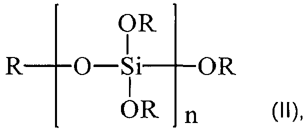 wobei
   n Werte im Bereich von 2 bis 100 annimmt und
   alle R unabhängig voneinander ausgewählt sind aus der Gruppe aus Wasserstoff, geradkettigen oder verzweigten, gesättigten oder ungesättigten, substituierten oder unsubstituierten C₁₋₆-Kohlenwasserstoffresten und Duftstoff-Alkohol-Resten, unter der Maßgabe, dass mindestens ein R für einen Duftstoff-Alkohol-Rest steht,
   oder Gemische dieser Verbindungen.

Bevorzugt werden solche Verbindungen der allgemeinen Formel (I) in den erfindungsgemäßen Kombinationen eingesetzt, bei denen R¹, R², R³, R⁴ unabhängig voneinander für Reste stehen, die in einer Verbindung der allgemeinen Formel R¹-C(=O)-R² oder R³-C(=O)-R⁴ je einen Duftstoff-Aldehyd mit mindestens 6 Kohlenstoffatomen ergeben.

In einer besonderen Ausführungsform der vorliegenden Erfindung werden solche Verbindungen der allgemeinen Formel (I) in den erfindungsgemäßen Kombinationen eingesetzt, bei denen maximal in einem der Strukturelemente -CR¹R² bzw. -CR³R⁴ Reste R¹ und R² bzw. R³ und R⁴ vorliegen, die in einer Verbindung der allgemeinen Formel R¹-C(=O)-R² bzw. R³-C(=O)-R⁴ ein Duftstoff-Keton ergeben.

Bevorzugte Reste R⁶ sind Methyl-, Ethyl-, und Hydroxymethylreste. R⁵ und R⁷ sind bevorzugt Wasserstoff oder ein C₁₋₆-Alkylrest, vorzugsweise C₁₋₃-Alkylrest. Besonders bevorzugt sind R⁵ und R⁷ Wasserstoff oder ein Methyl- oder Ethylrest, insbesondere Wasserstoff.
In insbesondere bevorzugten Verbindungen der allgemeinen Formel (I) bedeuten R², R⁴, R⁵, R⁶, R⁷ Wasserstoff und R¹ und R³ jeweils einen C₅₋₂₄-Kohlenwasserstoffrest.

Bevorzugt werden solche Verbindungen der allgemeinen Formel (I) in den erfindungsgemäßen Kombinationen eingesetzt, in denen die Reste R¹, R², R³ und R⁴ in einer Verbindung der allgemeinen Formel R¹-C(=O)-R² oder R³-C(=O)-R⁴ einen Duftstoff-Aldehyd oder ein Duftstoff-Keton ergeben, welches ausgewählt ist aus der Liste aus den Jasmonen; lononen, Damasconen und Damascenonen, Menthon, Carvon, Iso-E-Super, Methyl-heptenon, Melonal, Cymal; Ethylvanillin; Helional; Hydroxycitronellal; Koavon; Methyl-nonyl-acetaldehyd; Phenylacetaldehyd; Undecylenaldehyd; 3-Dodecen-l-al, alpha-n-Amylzimtaldehyd, Benzaldehyd, 3-(4-tert-Butylphenyl)-propanal, 2-Methyl-3-(para-methoxyphenylpropanal), 2-Methyl-4-(2,6,6-trimethyl-2(1)-cyclohexen-1-yl)butanal, 3-Phenyl-2-propenal, cis-/trans-3,7-Dimethyl-2,6-octadien-l-al, 3,7-Dimethyl-6-octen-l-al, [(3,7-Dimethyl-6-octenyl)oxy]acetaldehyd, 4-Isopropylbenzyaldehyd, 2,4-Dimethyl-3-cyclohexen-1-carboxyaldehyd, 2-Methyl-3-(isopropylphenyl)propanal, Decylaldehyd, 2,6-Dimethyl-5-heptenal; alpha-n-Hexylzimtaldehyd, 7-Hydroxy-3,7-dimethyl octanal, Undecenal, 2,4,6-Trimethyl-3-cyclohexene-l-carboxaldehyd, 1-Dodecanal, 2,4-Dimethylcyclohexene-3-carboxaldehyd, 4-(4-Hydroxy-4-methyl pentyl)-3-cylohexene-l-carboxaldehyd, 2-Methyl undecanal, 2-Methyl decanal, 1-nonanal, 1-Octanal, 2,6,10-Trimethyl-5,9-undecadienal, 2-Methyl-3-(4-tertbutyl)propanal, Dihydrozimtaldehyd, 3,7-Dimethyloctan-1-al, 1-Undecanal, 10-Undecen-1-al, 4-Hydroxy-3-methoxybenzaldehyd, trans-4-decenal, 2,6-Nonadienal, para-Tolylacetaldehyd; 3,7-Dimethyl-2-methylene-6-octenal, 2-Methyloctanal, alpha-Methyl-4-(l-methylethyl)benzeneacetaldehyd, 2-methyl-3-phenyl-2-propen-1-al, 3,5,5-Trimethylhexanal, 3-Propyl-bicyclo[2.2.1]hept-5-ene-2-carbaldehyd, 9-Decenal, 3-Methyl-5-phenyl-1-pentanal, Methylnonylacetaldehyd, Citral, 1-Decanal, Florhydral, 2,4-Dimethyl-3-cyclohexen-1-carboxaldehyd und/oder Heliotropin. Diese Duftstoff-Aldehyde und/oder Duftstoff-Ketone zeigen besonders gute Ergebnisse bei der Bekämpfung und Überdeckung von Schlechtgerüchen.

Bevorzugt werden solche Kieselsäureester der allgemeinen Formel (II) in den erfindungsgemäßen Kombinationen eingesetzt, bei welchen mindestens 10 mol-%, vorzugsweise mindestens 20 mol-% und insbesondere bevorzugt sogar mehr als 40 mol-% der Reste R Duftstoff-Alkohol-Reste sind. Besonders bevorzugt ist, wenn diese Duftstoff-Alkohol-Reste ausgewählt sind aus der Gruppe der Reste folgender Duftstoff-Alkohole: 10-Undecen-1-ol, 2,6-Dimethylheptan-2-ol, 2-Methyl-butanol, 2-Methylpentanol, 2-Phenoxyethanol, 2-Phenylpropanol, 2-tert.-Butycyclohexanol, 3,5,5-Trimethylcyclohexanol, 3-Hexanol, 3-Methyl-5-phenyl-pentanol, 3-Octanol, 3-Phenyl-propanol, 4-Heptenol, 4-Isopropylcyclohexanol, 4-tert.-Butycyclohexanol, 6,8-Dimethyl-2-nona-nol, 6-Nonen-1-ol, 9-Decen-1-ol, α-Methylbenzylalkohol, α-Terpineol, Amylsalicylat, Benzylalkohol, Benzylsalicylat, β-Terpineol, Butylsalicylat, Citronellol, Cyclohexylsalicylat, Decanol, Di-hydromyrcenol, Dimethylbenzylcarbinol, Dimethylheptanol, Dimethyloctanol, Ethylsalicylat, Ethylvanillin, Eugenol, Farnesol, Geraniol, Heptanol, Hexylsalicylat, Isoborneol, Isoeugenol, Isopulegol, Linalool, Menthol, Myrtenol, n-Hexanol, Nerol, Nonanol, Octanol, p-Menthan-7-ol, Phenylethylalkohol, Phenol, Phenylsalicylat, Tetrahydrogeraniol, Tetrahydrolinalool, Thymol, trans-2-cis-6-Nonadicnol, trans-2-Nonen-1-ol, trans-2-Octenol, Undecanol, Vanillin, Champiniol, Hexenol und/oder Zimtalkohol. Diese Duftstoff-Alkohole zeigen besonders gute Ergebnisse bei der Bekämpfung und Überdeckung von Schlechtgerüchen.

Besonders hervorragende Ergebnisse bei der Bekämpfung und Überdeckung von Schlechtgerüchen konnten beim Einsatz von Verbindungen der allgemeinen Formel (I) mit Resten, die einen der oben aufgeführten, bevorzugten Duftstoff-Aldehyd und/oder Duftstoff-Keton ergeben in Kombination mit einem Kieselsäureester der allgemeinen Formel (II) mit Resten, die einen der oben aufgeführten, bevorzugten Duftstoff-Alkohol ergeben, erzielt werden.

Des Weiteren können die erfindungsgemäßen Kombinationen aus Verbindungen der allgemeinen Formel (I) und (II) Mischungen mit Verbindungen der allgemeinen Formel (III) 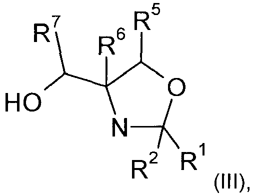 wobei
R¹, R² unabhängig voneinander für Reste stehen, die in einer Verbindung der allgemeinen Formel R¹-C(=O)-R² einen Duftstoff-Aldehyd mit mindestens 6 Kohlenstoffatomen oder ein Duftstoff-Keton mit mindestens 6 Kohlenstoffatomen ergeben,
R⁵, R⁶, R⁷ unabhängig voneinander für H oder einen Kohlenwasserstoff-Rest stehen, der acyclisch oder cyclisch, substituiert oder unsubstituiert, verzweigt oder unverzweigt sowie gesättigt oder ungesättigt sein kann,
oder Gemische dieser Verbindungen, enthalten.

Ein weiterer Gegenstand der vorliegenden Erfindung ist die Verwendung der erfindungsgemäßen Kombinationen der Verbindung der allgemeinen Formel (I) und (II) oder Mischungen derer mit Verbindungen der allgemeinen Formel (III) als Duftstoffvorläufer. Die erfindungsgemäßen Duftstoffvorläufer setzen dabei vorzugsweise die darin derivatisierten Duftstoffe durch Hydrolyse nach und nach wieder frei.

Die erfindungsgemäßen Verbindungen und Mittel zeigen unter Umgebungsbedingungen eine gute hydrolytische Spaltbarkeit. Sie weisen zudem eine hohe Lagerstabilität in alkalischer Umgebung auf, wie sie beispielsweise in Waschmitteln und Geschirrreinigungsmitteln anzutreffen ist.

Aufgrund der hervorragenden Eignung der erfindungsgemäßen Verbindungen zum Einsatz in Wasch- und Reinigungsmitteln ist die Verwendung der erfindungsgemäßen Duftstoffvorläufer, in flüssigen oder festen Wasch- und Reinigungsmitteln, insbesondere bevorzugt als Duftstoff, ein weiterer Gegenstand der vorliegenden Erfindung.

Ebenso eignen sich die erfindungsgemäßen Duftstoffvorläufer hervorragend zum Einsatz in Kosmetika (kosmetischen Mitteln), daher ist ein weiterer Gegenstand der vorliegenden Erfindung die Verwendung der erfindungsgemäßen Duftstoffvorläufer in Kosmetika (kosmetischen Mitteln) zur Haut- und Haarbehandlung insbesondere bevorzugt als Duftstoff.

In einer besonderen Ausführungsform, setzen die beanspruchten Kombinationen Duftstoff-Aldehyde und Duftstoff-Alkohole als Duftstoffe frei. In einer weiteren besonderen Ausführungsform, setzen die beanspruchten Kombinationen Duftstoff-Aldehyde, Duftstoff-Ketone und Duftstoff-Alkohole als Duftstoffe frei. Bevorzugt werden die erfindungsgemäßen Kombinationen zusammen mit anderen Duftstoffen verwendet.

Bevorzugt sind die erfindungsgemäßen Kombinationen der Verbindung der allgemeinen Formel (I) und (II) oder Mischungen derer mit Verbindungen der allgemeinen Formel (III) in Mengen von weniger als 5 Gew.-%, vorzugsweise weniger als 2 Gew.-%, insbesondere weniger als 1 Gew.-%, jeweils bezogen auf die Gesamtmenge der Mittel, in den Mitteln, bevorzugt Wasch- oder Reinigungsmittel, Weichspüler oder Kosmetika, enthalten.

Ein weiterer Gegenstand der vorliegenden Erfindung ist ein Verfahren zur Verlängerung des Duftempfindens von Mitteln, bevorzugt Wasch- oder Reinigungsmitteln, Weichspülern oder Kosmetika, oder mit diesen behandelten festen Oberflächen, wobei den Mitteln, bevorzugt Wasch- oder Reinigungsmitteln, Weichspülern oder Kosmetika, Kombinationen der Verbindung der allgemeinen Formel (I) und (II) oder Mischungen derer mit Verbindungen der allgemeinen Formel (III) zugesetzt werden. Vorzugsweise werden die Duftstoffe dabei durch Hydrolyse wieder freigesetzt.

Verfahren zum Abbau von Schlechtgerüchen durch Einsatz einer Kombination aus
(a) 1-Aza-3,7-dioxabicyclo[3.3.0]octan-Verbindung der allgemeinen Formel (I) 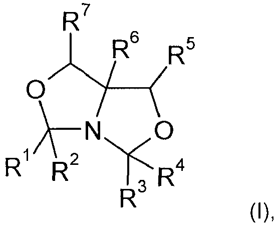 wobei
   R¹, R², R³, R⁴ unabhängig voneinander für Reste stehen, die in einer Verbindung der allgemeinen Formel R¹-C(=O)-R² oder R³-C(=O)-R⁴ einen Duftstoff-Aldehyd mit mindestens 6 Kohlenstoffatomen oder ein Duftstoff-Keton mit mindestens 6 Kohlenstoffatomen ergeben, R⁵, R⁶, R⁷ unabhängig voneinander für H oder einen Kohlenwasserstoff-Rest stehen, der acyclisch oder cyclisch, substituiert oder unsubstituiert, verzweigt oder unverzweigt sowie gesättigt oder ungesättigt sein kann
   oder Gemische dieser Verbindungen und
(b) einem Kieselsäureester der allgemeinen Formel (II) 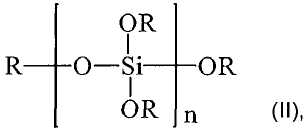 wobei
   n Werte im Bereich von 2 bis 100 annimmt und
   alle R unabhängig voneinander ausgewählt sind aus der Gruppe aus Wasserstoff, geradkettigen oder verzweigten, gesättigten oder ungesättigten, substituierten oder unsubstituierten C₁₋₆-Kohlenwasserstoffresten und Duftstoff-Alkohol-Resten, unter der Maßgabe, dass mindestens ein R für einen Duftstoff-Alkohol-Rest steht,
   oder Gemische dieser Verbindungen.

Öllösliche substituierte mono- und bicyclische Oxazolidine, die als Additive beispielsweise in Automatikgetriebeflüssigkeiten eingesetzt werden, sind aus der US 4,277,353 bekannt. Beispielhaft werden Umsetzungsprodukte von gegebenenfalls substituierten 2-Amino-1,3-propandiolen mit Paraformaldehyd und Isobutyraldehyd beschrieben. Die Derivatisierung von Duftstoff-Aldehyden oder Duftstoff-Ketonen ist jedoch nicht erwähnt. Gemäß einer Ausführungsform der vorliegenden Erfindung sind die folgenden Verbindungen ausgenommen: 1-Aza-3,7-dioxa-5-methyl-bicyclo[3.3.0]octan, 1-Aza-3,7-dioxa-5-ethyl-bicyclo[3.3.0]octan, 1-Aza-3,7-dioxabicyclo[3.3.0]octan und 1-Aza-3,7-dioxa-2,8-diisopropyl-5-ethyl-bicyclo[3.3.0]octan. Ferner sind gemäß einer Ausführungsform der vorliegenden Erfindung R¹ und R³ keine C₁₋₃₀-Hydrocarbylreste, sofern R² und R⁴ und R⁵ und R⁷ Wasserstoff sind und R⁶ Wasserstoff, Methyl oder Ethyl bedeutet. Ferner sind gemäß einer Ausführungsform der Erfindung Verbindungen ausgeschlossen, in denen in dem Strukturelement-CR¹R² der Rest R¹ ein C₁₋₃₀-Hydrocarbylrest ist und R² Wasserstoff ist, und in dem Strukturelement -CR³R⁴ die Reste R³ und R⁴ jeweils C₁₋₇-Hydrocarbylreste darstellen.

Als Duftstoff-Aldehyde, Duftstoff-Ketone oder Duftstoff-Alkohole im Sinne dieser Anmeldung werden alle Riech- und Duftstoffe der chemischen Substanzklassen der Aldehyde, Ketone oder Alkohole verstanden, die typischerweise zur Herbeiführung eines angenehmen Duftempfindens eingesetzt werden.

In den Verbindungen der allgemeinen Formel (I) liegen Duftstoff-Aldehyde und/oder Duftstoff-Ketone vor, die mit 2-Amino-1,3-propandiolen und deren Derivaten umgesetzt sind. Die Duftstoff-Ketone können alle Ketone umfassen, die einen erwünschten Duft oder ein Frischeempfinden verleihen können. Es können auch Gemische unterschiedlicher Ketone eingesetzt werden. Beispielsweise kann das Keton ausgewählt sein aus der Gruppe, bestehend aus Buccoxim, Iso-jasmon, Methyl-beta- naphthyl-keton, Moschus-indanon, Tonalid/Moschus plus, Alpha-Damascon, Beta-Damascon, Delta-Damascon, Iso-Damascon, Damascenon, Damarose, Methyl-dihydrojasmonat, Menthon, Carvon, Campher, Fenchon, Alpha-Ionen, Beta-Ionon, Dihydro-Beta-Ionon, Gamma-Methyl so genanntes Ionon, Fleuramon, Dihydrojasmon, Cis-Jasmon, Iso-E-Super, Methyl-cedrenylketon oder Methyl-cedrylon, Acetophenon, Methyl-acetophenon, Para-Methoxy-acetophenon, Methyl-beta-naphtyl-keton, Benzyl-aceton, Benzophenon, Para-Hydroxy-phenyl-butanon, Celery Keton oder Livescon, 6-Isopropyldecahydro-2-naphton, Dimethyl- octenon, Freskomenth, 4-(l-Ethoxyvinyl)-3,3,5,5,-tetramethyl-cyclohexanon, Methyl-heptenon, 2-(2-(4-Methyl-3-cyclohexen-1-yl)propyl)-cyclopentanon, 1-(p-Menthen-6(2)-yl)-1-propanon, 4-(4-Hydroxy-3-methoxyphenyl)-2-butanon, 2-Acetyl-3,3-dimethyl-norbornan, 6,7-Dihydro-1,1,2,3,3-pentamethyl-4(5H)-indanon, 4-Damascol, Dulcinyl or Cassion, Gelson, Hexalon, Isocyclemon E, Methyl-cyclocitron, Methyl-Lavendel-keton, Orivon, Para-tert-butyl-cyclohexanon, Verdon, Delphon, Muscon, Neobutenon, Plicaton, Velouton, 2,4,4,7-Tetramethyl-oct-6-en-3-on, Tetrameran, Hedion und Gemischen davon. Bevorzugt können die Ketone ausgewählt sein aus Alpha Damascon, Delta Damascon, Iso Damascon, Carvon, Gamma-Methyl-ionon, Iso-E-Super, 2,4,4,7-Tetramethyl-oct-6-en-3-on, Benzylaceton, Beta Damascon, Damascenon, Methyldihydrojasmonat, Methyl-cedrylon, Hedion und Gemischen davon.

Geeignete Duftstoff-Aldehyde können beliebige Aldehyde sein, die entsprechend der Duftstoff-Ketone einen gewünschten Duft oder eine Frischeempfinden vermitteln. Es kann sich wiederum um einzelne Aldehyde oder Aldehydgemische handeln. Geeignete Aldehyde sind beispielsweise Melonal, Triplal, Ligustral, Adoxal, Anisaldehyd, Cymal, Ethylvanillin, Florhydral, Helional, Heliotropin, Hydroxycitronellal, Koavon, Laurinaldehyd, Lyral, Methyl-nonyl-acetaldehyd, p,t-Bucinal, Phenylacetaldehyd, Undecylenaldehyd, Vanillin, 2,6,10-Trimethyl-9-undecenal, 3-Dodecen-l-al, alpha-n-Amylzimtaldehyd, 4-Methoxybenzaldehyd, Benzaldehyd, 3-(4-tert-Butylphenyl)-propanal, 2-Methyl-3-(para-methoxyphenylpropanal), 2-Methyl-4-(2,6,6-trimethyl-2(1)-cyclohexen-1-yl)butanal, 3-Phenyl-2-propenal, cis-/trans-3,7-Dimethyl-2,6-octadien-l-al, 3,7-Dimethyl-6-octen-l-al, [(3,7-Dimethyl-6-octenyl)oxy]acetaldehyd, 4-Isopropylbenzyaldehyd, 1,2,3,4,5,6,7,8-octahydro-8,8-Dimethyl-2-naphthaldehyd, 2,4-Dimethyl-3-cyclohexen-1-carboxyaldehyd, 2-Methyl-3-(isopropylphenyl)propanal, Decyl aldehyd, 2,6-Dimethyl-5-heptenal; 4-(tricyclo[5.2. 1.0 (2,6)]-decylidene-8)-butanal, Octahydro-4,7-methano-IH-indenecarboxaldehyd, 3-Ethoxy-4-hydroxybenzaldehyd, para-Ethyl-alpha, alpha-dimethylhydrozimtaldehyd, alpha-Methyl-3,4-(methylenedioxy)-hydrozimtaldehyd, 3,4-Methylenedioxybenzaldehyd, alpha-n-Hexylzimtaldehyd,
m-Cymene-7-carboxaldehyd, alpha-Methylphenylacetaldehyd, 7-Hydroxy-3,7-dimethyl octanal, Undecenal, 2,4,6-Trimethyl-3-cyclohexene-l-carboxaldehyd, 4-(3)(4-Methyl-3-pentenyl)-3-cyclohexen-carboxaldehyd, 1-Dodecanal, 2,4-Dimethyl cyclohexene-3-carboxaldehyd, 4-(4-Hydroxy-4-methyl pentyl)-3-cylohexene-l-carboxaldehyd, 7-Methoxy-3,7-dimethyloctan-1-al, 2-Methyl undecanal, 2-Methyl decanal,1-nonanal, 1-Octanal, 2,6,10-Trimethyl-5,9-undecadienal, 2-Methyl-3-(4-tert-butyl)propanal, Dihydrozimtaldehyd,1-methyl-4-(4-methyl-3-pentenyl)-3-cyclohexene-1-carboxaldehyd, 5 oder 6 Methoxyhexahydro-4,7-methanoindan-1 oder 2-carboxyaldehyd, 3,7-Dimethyloctan-1-al, 1-Undecanal,10-Undecen-1-al, 4-Hydroxy-3-methoxybenzaldehyd, 1-Methyl-3-(4-methylpentyl)-3-cyclohexenecarboxyaldehyd, 7-Hydroxy-3,7-dimethyl-octanal, trans-4-decenal, 2,6-Nonadienal, para-Tolylacetaldehyd, 4-Methylphenylacetal-dehyd, 2-Methyl-4-(2,6,6-trimethyl-1-cyclohexen-1-yl)-2-butenal, ortho-Methoxyzimtaldehyd, 3,5,6-Trimethyl-3-cyclohexenecarboxaldehyd, 3,7-Dimethyl-2-methylene-6-octenal, Phenoxyacetaldehyd, 5,9-Dimethyl-4,8-decadienal, Peony aldehyd (6,10-dimethyl-3-oxa-5,9-undecadien-1-al), Hexahydro-4,7-methanoindan-1-carboxaldehyd, 2-Methyl octanal, alpha-Methyl-4-(l-methylethyl) benzeneacetaldehyd, 6,6-Dimethyl-2-norpinene-2-propionaldehyd, para Methyl phenoxy acetaldehyd, 2-methyl-3-phenyl-2-propen-1-al, 3,5,5-Trimethylhexanal, Hexahydro-8,8-dimethyl-2-naphthal-dehyd, 3-Propyl-bicyclo [2.2.1]-hept-5-ene-2-carbaldehyd, 9-Decenal, 3-Methyl-5-phenyl-1-pentanal, Methylnonyl acetaldehyd, 1-p-Menthene-q-carboxaldehyd, Citral oder Gemische davon, Lilial citral, 1-Decanal, Florhydral, 2,4-Dimethyl-3-cyclohexen-1-carboxaldehyd, bevorzugte Aldehyde können ausgewählt sein aus cis/trans-3,7-Dimethyl-2,6-octadien-l-al, Heliotropin, 2,4,6-Trimethyl-3-cyclohexene-l-carboxaldehyd, 2,6-Nonadienal, alpha-n-Amylzimtaldehyd, alpha-n-Hexylzimtaldehyd, p-tert Bucinal, Lyral, Cymal, Methylnonylacetaldehyd, trans-2-Nonenal, Lilial, trans-2-Nonenal und Gemische davon.

Wie vorstehend beispielhaft ausgeführt, können die Duftstoff-Aldehyde und Duftstoff-Ketone eine aliphatische, cycloaliphatische, aromatische, ethylenisch ungesättigte Struktur oder eine Kombination dieser Strukturen aufweisen. Es können ferner weitere Heteroatome oder polycyclische Strukturen vorliegen. Die Strukturen können geeignete Substituenten wie Hydroxyl- oder Aminogruppen aufweisen.

Die Kombination enthält durch die Verbindungen der allgemeinen Formel (I) ferner einen Duftstoffaldehyd und/oder ein Duftstoffketon. Dies bedeutet, dass die Kombination nur einen Typ Duftstoff-aldehyd oder nur einen Typ Duftstoffketon enthalten kann. Davon umfasst ist aber auch, dass die Kombination einen Typ Duftstoffaldehyd und einen Typ Duftstoffketon enthalten kann. Ferner ist umfasst, dass die Kombinationen mehrere Typen Duftstoffaldehyd oder mehrere Typen Duftstoffketon enthalten kann. Ebenso ist es möglich, dass die Kombination mehrere Typen Duftstoffaldehyd und einen Typ Duftstoffketon oder mehrere Typen Duftstoffaldehyd und mehrere Typen Duftstoffketon enthält. In einer weiteren Ausführungsform enthält die Kombination mehrere Typen Duft stoffketon und einen Typ Duftstoffaldehyd oder mehrere Typen Duftstoffketon und mehrere Typen Duftstoffaldehyde.

In den Verbindungen der allgemeinen Formel (II) liegen Duftstoff-Alkohole vor, die mit Kieselsäuren und deren Derivaten umgesetzt sind.

Oligokieselsäureester niederer Alkohole sind kommerziell erhältlich, wobei üblicherweise Methanol, Ethanol, n-Propanol, iso-Propanol, n-Butanol, iso-Butanol und tert-Butanol zur Veresterung eingesetzt wurden. Die Darstellung nicht vollständig mit Duftstoffalkoholen umgeesterter Oligokieselsäureester führt zu Kieselsäureester-Mischungen in denen ein Teil der Reste R ausgewählt ist aus der Gruppe Methyl, Ethyl, n-Propyl, iso-Propyl, n-Butyl, iso-Butyl und tert-Butyl. Solche Verbindungen sind im Rahmen der vorliegenden Erfindung bevorzugt.

Erfindungsgemäß sind solche Oligokieselsäureester, die mindestens einen Rest R aus der Gruppe der Duftstoff-Alkohol-Reste enthalten.

Die Herstellung der genannten Verbindungen gelingt durch einfache Umesterung von Oligokieselsäureestern niederer Alkohole mit Duftstoffalkoholen, wobei sowohl einzelne Duftstoffalkohole als auch Duftstoffalkoholgemische eingesetzt werden können. Je nach Reaktionszeit und -bedingungen werden die niederen Alkohole abgespalten und die Duftstoff- bzw. Biozidalkohole gebunden, wobei die Alkohole entlang von Si-O-Si-Ketten oder -Ringen leichter ausgetauscht werden als die terminalen Alkohole. Üblicherweise werden als Edukte die handelsüblichen Kieselsäureester eingesetzt. Hier ist insbesondere der Ethanol-Ester zu nennen. Die Umesterung kann dabei ausschließlich durch Temperaturerhöhung und Abdestillation der leichtflüchtigen Nebenprodukte gesteuert werden. bevorzugt ist es jedoch, wenn zur Umesterung Katalysatoren eingesetzt werden. Es handelt sich dabei üblicherweise um Lewis-Säuren, vorzugsweise um Aluminiumtetraisopropylat, Titantetraisopropylat, Siliciumtetrachlorid oder basische Katalysatoren oder auch um Zubereitungen wie beispielsweise aus Aluminiumoxid mit Kaliumfluorid. Die so gebildeten oligomeren Kieselsäureester weisen dann zumindest teilweise Duftstoffalkoholreste auf. Üblicherweise enthalten die resultierenden Ester jedoch auch noch Reste niederer Alkohole. Soweit bei der Herstellung der Kieselsäureester geringe Mengen Wasser oder anderer Wasserstoff-acider Verbindungen anwesend sind, findet auch Austausch von Alkohol-Resten gegen OH-Gruppen statt. Dementsprechend enthalten die erfindungsgemäßen Kieselsäureester-Mischungen üblicherweise als Rest R teilweise auch Wasserstoff.

Die vollständig umgeesterten Oligokieselsäureester sind im Rahmen der vorliegenden Erfindung besonders bevorzugt. Insbesondere ist es dabei bevorzugt, wenn diese Ester nur einen einzigen Rest R enthalten, d.h. nur einen einzigen Duftstoffalkohol enthalten.

Die Oligomerisierungsgrade "n" der erfindungsgemäßen Kieselsäureester liegen zwischen 2 und 100, bevorzugt zwischen 2 und 20. In bevorzugten Verbindungen nimmt n Werte zwischen 2 und 15, vorzugsweise zwischen 2 und 12 und insbesondere zwischen 3 und 10 unter besonderer Bevorzugung der Werte 4, 5, 6, 7 und 8, an.

Unter dem Begriff Duftstoff-Alkohole werden im Rahmen der vorliegenden Erfindung Duftstoffe verstanden, die über freie Hydroxylgruppen verfügen, welche veresterbar sind, unabhängig davon, wie das Molekül weiter aufgebaut ist. So lassen sich auch Salicylsäureester als Duftstoffalkohole einsetzen. Aus der großen Gruppe der Duftstoffalkohole lassen sich bevorzugte Vertreter nennen, so dass im Rahmen der vorliegenden Erfindung Kieselsäureester bevorzugt sind, in denen jedes R unabhängig voneinander ausgewählt ist aus der Gruppe der Reste folgender Duftstoffalkohole: 10-Undecen-1-ol, 2,6-Dimethylheptan-2-ol, 2-Methylbutanol, 2-Methylpentanol, 2-Phenoxyethanol, 2-Phenyl-propanol, 2-tert-Butycyclohexanol, 3,5,5-Trimethylcyclohexanol, 3-Hexanol, 3-Methyl-5-phenylpentanol, 3-Octanol, 3-Phenylpropanol, 4-Heptenol, 4-Isopropylcyclo-hexanol, 4-tert-Butycyclohexanol, 6,8-Dimethyl-2-nonanol, 6-Nonen-1-ol, 9-Decen-1-ol, α-Methylbenzylalkohol, α -Terpineol, Amylsalicylat, Benzylalkohol, Benzylsalicylat, β-Terpineol, Butylsalicylat, Citronellol, Cyclohexylsalicylat, Decanol, Dihydromyrcenol, Dimethylbenzylcarbinol, Dimethylheptanol, Dimethyloctanol, Ethylsalicylat, Ethylvanillin, Eugenol, Farnesol, Geraniol, Heptanol, Hexylsalicylat, Isoborneol, Isoeugenol, Isopulegol, Linalool, Menthol, Myrtenol, n-Hexanol, Nerol, Nonanol, Octanol, para-Menthan-7-ol, Phenylethylalkohol, Phenol, Phenylsalicylat, Tetrahydrogeraniol, Tetrahydrolinalool, Thymol, trans-2-cis-6-Nonadicnol, trans-2-Nonen-1-ol, trans-2-Octenol, Undecanol, Vanillin, Zimtalkohol, Citronellol, Eugenol, Farnesol, Thymol und Geraniol. Weitere Biozidalkohole sind Phenoxyethanol, 1,2-Propylenglykol, Glycerin, Citronensäure und deren Ester, Milchsäure und deren Ester, Salicylsäure und deren Ester, 2-Benzyl-4-chlorphenol und 2,2'-Methylen-bis-(6-brom-4-chlorphenol).

Bevorzugt werden Phenylethylkieselsäureester, Geranylkieselsäureester, Citronellylkieselsäureester, Cynamylkieselsäureester, Hexenylkieselsäureester, Nonadienylkieselsäureester, Octenylkieselsäureester oder Mischungen aus zwei oder mehr dieser Kieselsäureester eingesetzt.

Die erfindungsgemäßen Kombinationen der allgemeinen Formeln (I) und (II) werden erfindungsgemäß als Duftstoffvorläufer eingesetzt. Der Begriff "Duftstoffvorläufer" beschreibt dabei Derivate von Duftstoff-Aldehyden, Duftstoff-Alkoholen und Duftstoff-Ketonen, die unter Umgebungsbedingungen die ursprünglichen Duftstoff-Aldehyde, Duftstoff-Alkohole und Duftstoff-Ketone freisetzen. Umgebungsbedingungen sind dabei die typischen Umgebungsbedingungen im menschlichen Lebensraum bzw. die auf der menschlichen Haut anzutreffenden Bedingungen. Unter Umgebungsbedingungen zerfallen die Verbindungen der allgemeinen Formel (I) in Umkehr des Herstellungsverfahrens langsam unter Freisetzung der ursprünglichen Duftstoff-Aldehyde und/oder Duftstoff-Ketone. Die chemisch gebundenen Duftstoff-Aldehyde und Duftstoff-Ketone werden an der Bindungsstelle gespalten, wodurch die Duftstoffe wieder freigesetzt werden.

Die erfindungsgemäßen Duftstoffvorläufer können als alleiniger Duftstoff eingesetzt werden, es ist aber auch möglich, Duftstoffgemische einzusetzen, die lediglich zu einem Teil aus dem erfindungsgemäßen Duftstoffvorläufer bestehen. So können insbesondere Duftstoffgemische eingesetzt werden, die 1 bis 50 Gew.-%, vorzugsweise 5 bis 40 Gew.-% und insbesondere maximal 30 Gew.-%, bezogen auf die Gesamtmenge an eingesetzten Duftstoffen aus freien Duftstoffen und Duftstoffvorläufern, an erfindungsgemäßen Duftstoffvorläufer enthalten. In anderen Ausführungsformen, bei denen insbesondere die verzögerte Duftwirkung der Träger-gebundenen Form genutzt werden soll, werden bei der erfindungsgemäßen Verwendung vorteilhaft mindestens 30 Gew.-%, vorzugsweise mindestens 40 Gew.-% und insbesondere mindestens 50 Gew.-% des insgesamt in einem Mittel enthaltenen Parfüms über die erfindungsgemäßen Duftstoffvorläufer in das Mittel eingebracht, während die restlichen 70 Gew.-%, vorzugsweise 60 Gew.-% und insbesondere 50 Gew.-% des insgesamt im Mittel enthaltenen Parfüms auf übliche Weise aufgesprüht oder anders in das Mittel eingebracht werden. Die erfindungsgemäße Verwendung kann also vorteilhaft dadurch gekennzeichnet sein, dass die erfindungsgemäßen Duftstoffvorläufer zusammen mit anderen Duftstoffen eingesetzt werden.

Die Duftstoffe, die auf herkömmliche Weise in die Mittel inkorporiert werden können, sind keinerlei Beschränkungen unterworfen. So können als Parfümöle bzw. Duftstoffe einzelne Duftstoffverbindungen natürlichen oder synthetischen Ursprungs, z.B. vom Typ der Ester, Ether, Aldehyde, Ketone, Alkohole und Kohlenwasserstoffe verwendet werden. Duftstoffverbindungen vom Typ der Ester sind z. B. Benzylacetat, Phenoxyethylisobutyrat, p-tert-Butylcyclohexylacetat, Linalylacetat, Dimethylbenzylcarbinylacetat (DMBCA), Phenylethylacetat, Benzylacetat, Ethylmethylphenylglycinat, Allylcyclohexylpropionat, Styrallylpropionat, Benzylsalicylat, Cyclohexylsalicylat, Floramat, Melusat und Jasmacyclat. Zu den Ethern zählen beispielsweise Benzylethylether und Ambroxan, zu den Aldehyden z.B. die linearen Alkanale mit 8 bis 18 C-Atomen, Citral, Citronellal, Citronellyloxyacetaldehyd, Cyclamenaldehyd, Lilial und Bourgeonal, zu den Ketonen z.B. die Jonone, α-Isomethylionon und Methylcedrylketon, zu den Alkoholen Anethol, Citronellol, Eugenol, Geraniol, Linalool, Phenylethylalkohol und Terpineol, zu den Kohlenwasserstoffen gehören hauptsächlich Terpene wie Limonen und Pinen. Bevorzugt werden jedoch Mischungen verschiedener Duftstoffe verwendet, die gemeinsam eine ansprechende Duftnote erzeugen.

Solche Parfümöle können auch natürliche Duftstoffgemische enthalten, wie sie aus pflanzlichen Quellen zugänglich sind, z.B. Pine-, Citrus-, Jasmin-, Patchouly-, Rosen- oder Ylang-Ylang-Öl. Ebenfalls geeignet sind Muskateller-Salbeiöl, Kamillenöl, Nelkenöl, Melissenöl, Minzöl, Zimtblätteröl, Lindenblütenöl, Wacholderbeeröl, Vetiveröl, Olibanumöl, Galbanumöl und Labdanumöl sowie Orangenblütenöl, Neroliöl, Orangenschalenöl und Sandelholzöl.

Weitere herkömmliche Duftstoffe, die im Rahmen der vorliegenden Erfindung einsetzbar sind, sind beispielsweise die ätherischen Öle wie Angelikawurzelöl, Anisöl, Arnikablütenöl, Basilikumöl, Bayöl, Champacablütenöl, Edeltannenöl, Edeltannenzapfenöl, Elemiöl, Eukalyptusöl, Fenchelöl, Fichtennadelöl, Galbanumöl, Geraniumöl, Gingergrasöl, Guajakholzöl, Gurjunbalsamöl, Helichrysumöl, Ho-Öl, Ingweröl, Irisöl, Kajeputöl, Kalmusöl, Kamillenöl, Kampferöl, Kanagaöl, Kardamomenöl, Kassiaöl, Kiefernnadelöl, Kopaïvabalsamöl, Korianderöl, Krauseminzeöl, Kümmelöl, Kuminöl, Lavendelöl, Lemongrasöl, Limetteöl, Mandarinenöl, Melissenöl, Moschuskörneröl, Myrrhenöl, Nelkenöl, Neroliöl, Niaouliöl, Olibanumöl, Origanumöl, Palmarosaöl, Patschuliöl, Perubalsamöl, Petitgrainöl, Pfefferöl, Pfefferminzöl, Pimentöl, Pine-Öl, Rosenöl, Rosmarinöl, Sandelholzöl, Sellerieöl, Spiköl, Sternanisöl, Terpentinöl, Thujaöl, Thymianöl, Verbenaöl, Vetiveröl, Wacholderbeeröl, Wermutöl, Wintergrünöl, Ylang-Ylang-Öl, Ysop-Öl, Zimtöl, Zimtblätteröl, Zitronellöl, Zitronenöl sowie Zypressenöl sowie Ambrettolid, Ambroxan, α-Amylzimtaldehyd, Anethol, Anisaldehyd, Anisalkohol, Anisol, Anthranilsäuremethylester, Acetophenon, Benzylaceton, Benzaldehyd, Benzoesäureethylester, Benzophenon, Benzylalkohol, Benzylacetat, Benzylbenzoat, Benzylformiat, Benzylvalerianat, Borneol, Bornylacetat, Boisambrene forte, α-Bromstyrol, n-Decylaldehyd, n-Dodecylaldehyd, Eugenol, Eugenolmethylether, Eukalyptol, Farnesol, Fenchon, Fenchylacetat, Geranylacetat, Geranylformiat, Heliotropin, Heptincarbonsäuremethylester, Heptaldehyd, Hydrochinon-Dimethylether, Hydroxyzimtaldehyd, Hydroxyzimtalkohol, Indol, Iron, Isoeugenol, Isoeugenolmethylether, Isosafrol, Jasmon, Kampfer, Karvakrol, Karvon, p-Kresolmethylether, Cumarin, p-Methoxyaceto-phenon, Methyl-n-amylketon, Methylanthranilsäuremethylester, p-Methylacetophenon, Methylchavikol, p-Methylchinolin, Methyl-β-naphthylketon, Methyl-n-nonylacetaldehyd, Methyl-n-nonylketon, Muskon, β-Naphtholethylether, β-Naphthol-methylether, Nerol, n-Nonylaldehyd, Nonylalkohol, n-Octylaldehyd, p-Oxy-Acetophenon, Pentadekanolid, β-Phenylethylalkohol, Phenylacetaldehyd-Dimethyacetal, Phenylessigsäure, Pulegon, Safrol, Salicylsäureisoamylester, Salicylsäuremethylester, Salicylsäurehexylester, Salicylsäurecyclohexylester, Santalol, Sandelice, Skatol, Terpineol, Thymen, Thymol, Troenan, γ-Undelacton, Vanillin, Veratrumaldehyd, Zimtaldehyd, Zimtalkohol, Zimtsäure, Zimtsäureethylester, Zimtsäurebenzylester, Diphenyloxid, Limonen, Linalool, Linalylacetat und - propionat, Melusat, Menthol, Menthon, Methyl-n-heptenon, Pinen, Phenylacetaldehyd, Terpinylacetat, Citral, Citronellal und Mischungen daraus.

Die erfindungsgemäßen Duftstoffvorläufer werden bevorzugt in Wasch- und Reinigungsmitteln, Weichspülern und Kosmetika eingesetzt. Es kann sich dabei um feste, gelförmige oder flüssige Formulierungen handeln, wobei feste Formulierungen in Pulver-, Granulat-, Tabletten- oder Tab-Form vorliegen können. Bei flüssigen Formulierungen kann es sich um Lösungen, Emulsionen oder Dispersionen handeln.

Waschmittel können dabei der manuellen oder maschinellen Wäsche von insbesondere Textilien dienen. Es kann sich um Wasch- oder Reinigungsmittel für den industriellen Bereich oder für den Haushaltsbereich handeln. Reinigungsmittel können beispielsweise zur Reinigung harter Oberflächen eingesetzt werden. Es kann sich dabei beispielsweise um Geschirrreinigungsmittel handeln, die zur manuellen oder maschinellen Reinigung von Geschirr eingesetzt werden. Es kann sich auch um übliche Industrie- oder Haushaltsreiniger handeln, mit denen harte Oberflächen wie Möbeloberflächen, Fliesen, Kacheln, Wand- und Bodenbeläge gereinigt werden. Bei Weichspülern handelt es sich insbesondere um Weichspüler, die zur Behandlung von Textilien bei oder nach der Wäsche eingesetzt werden. Bei Kosmetika kann es sich um Pasten, Salben, Cremes, Emulsionen, Lotionen sowie auch Lösungen, insbesondere alkoholische Lösungen handeln, die beispielsweise aus der Feinparfümerie bekannt sind. Die einzelnen Mittel können in jeder beliebigen geeigneten Form aufgebracht werden. Es kann sich beispielsweise um durch Sprühen aufzutragende Mittel handeln. Die erfindungsgemäßen Duftstoffvorläufer können darüber hinaus zur Überdeckung von Schlechtgerüchen eingesetzt werden, die beispielsweise in Kombination mit anderen Absorptionsmitteln eine gute Haftung an festen Oberflächen aufweisen.

Die Erfindung betrifft auch Wasch- oder Reinigungsmittel, Weichspüler oder Kosmetika, die die erfindungsgemäßen Verbindungen oder Mischungen enthalten. Dabei werden die Verbindungen oder Mischungen in zur Wirkung ausreichender Menge eingesetzt. Typischerweise werden in Endformulierungen, das heißt gebrauchsfertigen Wasch- oder Reinigungsmitteln, Weichspülern oder Kosmetika, Verbindungen der allgemeinen Formel (I) oder diese enthaltende Mischungen in Mengen von weniger als 5 Gew.-%, vorzugsweise weniger als 2 Gew.-%, insbesondere weniger als 1 Gew.-% eingesetzt. Typische Inhaltsmengen liegen im Bereich von 0,05 bis 0,5 Gew.-%, insbesondere 0,1 bis 0,2 Gew.-%. In der Feinparfümerie kann auch mit hohen Wirkstoffkonzentrationen von bis zu 40 Gew.-% an Duftstoffen gearbeitet werden.

Zusammensetzungen üblicher Wasch- oder Reinigungsmittel, Weichspüler und Kosmetika sind dem Fachmann bekannt. So können Wasch- und Reinigungsmittel und Weichspüler neben den erfindungsgemäßen Kombinationen der Verbindung der allgemeinen Formel (I) und (II) oder Mischungen derer mit Verbindungen der allgemeinen Formel (III) weitere übliche Inhaltsstoffe von Wasch- oder Reinigungsmitteln und Weichspüler enthalten, wie beispielsweise Tenside, Buildersubstanzen, Bleichmittel, andere Duftstoffe, Enzyme und andere Aktivstoffe, aber auch Desintegrationshilfsmittel, sogenannte Tablettensprengmittel, um den Zerfall hochverdichteter Tabletten oder Tabs zu erleichtern bzw. die Zerfallszeiten zu verkürzen.

Neben den genannten Bestandteilen können die erfindungsgemäßen Wasch- und Reinigungsmittel zusätzlich einen oder mehrere Stoffe aus der Gruppe der Bleichmittel, Bleichaktivatoren, Enzyme, pH-Stellmittel, Fluoreszenzmittel, Farbstoffe, Schauminhibitoren, Silikonöle, Antiredepositionsmittel, optischen Aufheller, Vergrauungsinhibitoren, Farbübertragungsinhibitoren, Korrosionsinhibitoren und Silberschutzmittel enthalten.

Erfindungsgemäß sind Wasch- und Reinigungsmittel auch Geschirrspülmittel. Grundsätzlich können die Mittel verschiedene Aggregatszustände aufweisen. In einer weiteren bevorzugten Ausführungsform handelt es sich bei den Weichspülern, Wasch- oder Reinigungsmitteln um flüssige oder gelförmige Mittel, insbesondere um Flüssigwaschmittel oder flüssige Geschirrspülmittel oder Reinigungsgele, wobei es sich insbesondere auch um gelförmige Reinigungsmittel für Spültoiletten handeln kann.

Die Erfindung wird durch die nachstehenden Beispiele näher erläutert.

### Beispiel 1

Zur Bestimmung des Abbaus von Schlechtgerüchen wurden eine Haushaltswaschmaschine (Miele W1735) mit 3 kg getragener Wäsche sowie 75 ml eines flüssigen Vollwaschmittels, welches 0,45 Gew.-% Parfüm (bezogen auf das gesamte flüssige Waschmittel) enthielt, beladen. Die Wäsche wurde bei 40°C gewaschen und dann für 4 Tage bei 20°C in der Waschtrommel liegen gelassen (V1).

Zehn olfaktorisch geschulte Personen haben die Wäsche frisch, nach 1 Tag und nach 4 Tagen abgerochen und die Intensität des Schlechtgeruchs auf einer Skala von 1 bis 10 (1 = nicht mehr wahrnehmbar bis 10 = extrem stark) bestimmt. Durch die in der Waschmaschine und im Waschwasser vorhandenen Bakterien und dem Eintrag durch die getragene Wäsche entsteht ein Schlechtgeruch.

In einem weiteren Waschversuch (V2) wurden zusätzlich zu dem Vollwaschmittel 0,6 Gew.-%, bezogen auf das Gesamtgewicht des Waschmittels, eines Kieselsäureestergemisches aus Phenylethylkieselsäureester, Geranylkieselsäureester und Citronellylkieselsäureester zudosiert.

In einem weiteren Waschversuch (V3) wurden zusätzlich zu dem Vollwaschmittel 0,6 Gew.-%, bezogen auf das Gesamtgewicht des Waschmittels, einer 1-Aza-3,7-dioxabicyclo[3.3.0]octan-Verbindung der allgemeinen Formel (I) mit Octanal als korrespondierendem Duftstoff-Aldehyd zudosiert.

In noch einem weiteren Waschversuch (E1) wurden zusätzlich zu dem Vollwaschmittel 0,3 Gew.-% eines Kieselsäureestergemisches aus Phenylethylkieselsäureester, Geranylkieselsäureester und Citronellylkieselsäureester und 0,3 Gew.-% eines einer 1-Aza-3,7-dioxabicyclo[3.3.0]octan-Verbindung der allgemeinen Formel (I) mit Octanal als korrespondierendem Duftstoff-Aldehyd zudosiert.

Die Ergebnisse sind in Tabelle 1 gezeigt.

**Tabelle 1: Intensität des Schlechtgeruchs**

| | V1 | V2 | V3 | E1 |
|---|---|---|---|---|
| Frisch | 2 | 2,2 | 2,4 | 2,3 |
| 1 Tag | 5,7 | 5,1 | 3,5 | 2,5 |
| 4 Tage | 8,4 | 7,6 | 4,5 | 2,6 |

Die Ergebnisse zeigen die deutlich verbesserte Leistung der erfindungsgemäßen Kombination bei der Bekämpfung und Überdeckung von Schlechtgerüchen durch Bakterien und dem Eintrag durch die getragene Wäsche, insbesondere über einen Zeitraum von mehreren Tagen.

### Beispiel 2

Ein Textil aus Baumwollfrottee wurde mit 100 mg des Schlechtgeruchs "Schweiss" (20 Gew.-% Octansäure, 20 Gew.-% Nonansäure, 20 Gew.-% 3-Methylbutansäure, 20 Gew.-% 2-Ethyl-2-hexensäure und 20 Gew.-% 3-Mercapto-1-hexanol) zwangsappliziert.

Anschließend wurde eine Haushaltswaschmaschine (Miele W1735) mit 3,5 kg Begleitwäsche sowie dem zwangsapplizierten Stofflappen beladen. Zusätzlich wurden 75 ml eines flüssigen Vollwaschmittels, welches 0,45 Gew.-% Parfüm (bezogen auf das gesamte flüssige Waschmittel) enthielt, zugegeben. Die Wäsche wurde bei 40°C gewaschen und dann für 4 Tage bei 20°C in der Waschtrommel liegen gelassen (V1).

Außerdem wurden Waschversuche durchgeführt, bei denen die Waschmaschine zusätzlich zu dem Vollwaschmittel
(V2) 0,6 Gew.-% eines Kieselsäureestergemisches aus Phenylethylkieselsäureester, Geranylkieselsäureester und Citronellylkieselsäureester,
(V3) 0,6 Gew.-% eines einer 1-Aza-3,7-dioxabicyclo[3.3.0]octan-Verbindung der allgemeinen Formel (I) mit Octanal als korrespondierendem Duftstoff-Aldehyd und
(E1) 0,3 Gew.-% eines Kieselsäureestergemisches aus Phenylethylkieselsäureester, Geranylkieselsäureester und Citronellylkieselsäureester und 0,3 Gew.-% einer 1-Aza-3,7-dioxabicyclo[3.3.0]octan-Verbindung der allgemeinen Formel (I) mit Octanal als korrespondierendem Duftstoff-Aldehyd
zudosiert wurden. Alle Gewichtsangaben sind bezogen auf das Gesamtgewicht des Waschmittels.

Die Ergebnisse sind in Tabelle 2 gezeigt.

**Tabelle 2: Intensität des Schlechtgeruchs**

| | V1 | V2 | V3 | E1 |
|---|---|---|---|---|
| Frisch | 9,1 | 8,6 | 5,3 | 4,8 |
| 1 Tag | 7,3 | 7,0 | 3,2 | 2,4 |
| 4 Tage | 6,5 | 6,1 | 2,7 | 2,2 |

Die Ergebnisse zeigen die deutlich verbesserte Leistung der erfindungsgemäßen Kombination bei der Bekämpfung und Überdeckung von Schlechtgerüchen, besonders Schweißgeruch, insbesondere über einen Zeitraum von mehreren Tagen.

## Patentansprüche

1. Kombination aus
(a) einer 1-Aza-3,7-dioxabicyclo[3.3.0]octan-Verbindung der allgemeinen Formel (I) 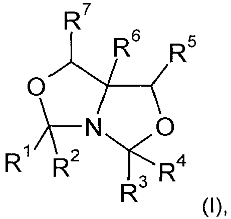 wobei
R¹, R², R³, R⁴ unabhängig voneinander für Reste stehen, die in einer Verbindung der allgemeinen Formel R¹-C(=O)-R² oder R³-C(=O)-R⁴ einen Duftstoff-Aldehyd mit mindestens 6 Kohlenstoffatomen oder ein Duftstoff-Keton mit mindestens 6 Kohlenstoffatomen ergeben,
R⁵, R⁶, R⁷ unabhängig voneinander für H oder einen Kohlenwasserstoff-Rest stehen, der acyclisch oder cyclisch, substituiert oder unsubstituiert, verzweigt oder unverzweigt sowie gesättigt oder ungesättigt sein kann
oder Gemische dieser Verbindungen und
(b) einem Kieselsäureester der allgemeinen Formel (II) 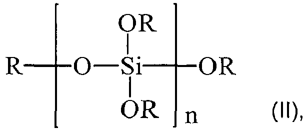 wobei
n Werte im Bereich von 2 bis 100 annimmt und
alle R unabhängig voneinander ausgewählt sind aus der Gruppe aus Wasserstoff, geradkettigen oder verzweigten, gesättigten oder ungesättigten, substituierten oder unsubstituierten C₁₋₆-Kohlenwasserstoffresten und Duftstoffalkoholresten, unter der Maßgabe, dass mindestens ein R für einen Duftstoffalkoholrest steht,
oder Gemische dieser Verbindungen.

2. Kombination nach Anspruch 1, wobei R¹, R², R³, R⁴ unabhängig voneinander für Reste stehen, die in einer Verbindung der allgemeinen Formel R¹-C(=O)-R² oder R³-C(=O)-R⁴ je einen Duftstoff-Aldehyd mit mindestens 6 Kohlenstoffatomen ergeben.

3. Kombination nach Anspruch 1, **dadurch gekennzeichnet, dass** maximal in einem der Strukturelemente -CR¹R² bzw. -CR³R⁴ Reste R¹ und R² bzw. R³ und R⁴ vorliegen, die in einer Verbindung der allgemeinen Formel R¹-C(=O)-R² bzw. R³-C(=O)-R⁴ ein Duftstoff-Keton ergeben.

4. Kombination nach einem der Ansprüche 1 bis 3, **dadurch gekennzeichnet, dass** in der Verbindung der allgemeinen Formel (I) die Reste R¹, R², R³ und R⁴ in einer Verbindung der allgemeinen Formel R¹-C(=O)-R² oder R³-C(=O)-R⁴ einen Duftstoff-Aldehyd oder ein Duftstoff-Keton ergeben, welches ausgewählt ist aus der Liste aus den Jasmonen; lononen, Damasconen und Damascenonen, Menthon, Carvon, Iso-E-Super, Methyl-heptenon, Melonal, Cymal, Ethylvanillin, Helional, Hydroxycitronellal, Koavon, Methyl-nonyl-acetaldehyd, Phenylacetaldehyd, Undecylenaldehyd, 3-Dodecen-l-al, alpha-n-Amylzimtaldehyd, Benzaldehyd, 3-(4-tert-Butylphenyl)-propanal, 2-Methyl-3-(para-methoxyphenylpropanal), 2-Methyl-4-(2,6,6-trimethyl-2(1)-cyclohexen-1-yl)butanal, 3-Phenyl-2-propenal, cis-/trans-3,7-Dimethyl-2,6-octadien-l-al, 3,7-Dimethyl-6-octen-l-al, [(3,7-Dimethyl-6-octenyl)oxy]acetaldehyd, 4-Isopropylbenzyaldehyd, 2,4-Dimethyl-3-cyclohexen-1-carboxyaldehyd, 2-Methyl-3-(isopropylphenyl)propanal, Decylaldehyd, 2,6-Dimethyl-5-heptenal, alpha-n-Hexylzimt-aldehyd, 7-Hydroxy-3,7-dimethyl octanal, Undecenal, 2,4,6-Trimethyl-3-cyclohexene-l-carboxaldehyd, 1-Dodecanal, 2,4-Dimethylcyclohexene-3-carboxaldehyd, 4-(4-Hydroxy-4-methyl pentyl)-3-cylohexene-l-carboxaldehyd, 2-Methyl undecanal, 2-Methyl decanal, 1-nonanal, 1-Octanal, 2,6,10-Trimethyl-5,9-undecadienal, 2-Methyl-3-(4-tertbutyl)propanal, Dihydrozimtaldehyd, 3,7-Dimethyloctan-1-al, 1-Undecanal, 10-Undecen-1-al, 4-Hydroxy-3-methoxybenzaldehyd, trans-4-decenal, 2,6-Nonadienal, para-Tolylacetaldehyd, 3,7-Dimethyl-2-methylene-6-octenal, 2-Methyloctanal, alpha-Methyl-4-(l-methylethyl)benzeneacetaldehyd, 2-methyl-3-phenyl-2-propen-1-al, 3,5,5-Trimethylhexanal, 3-Propyl-bicyclo[2.2.1]hept-5-ene-2-carbaldehyd, 9-Decenal, 3-Methyl-5-phenyl-1-pentanal, Methylnonylacetaldehyd, Citral, 1-Decanal, Florhydral, 2,4-Dimethyl-3-cyclohexen-1-carboxaldehyd, Heliotropin.

5. Kombination nach einem der Ansprüche 1 bis 4, **dadurch gekennzeichnet, dass** bei dem Kieselsäureester der allgemeinen Formel (II) mindestens 10 mol-%, vorzugsweise mindestens 20 mol-% und insbesondere bevorzugt sogar mehr als 40 mol-% der Reste R ausgewählt sind aus der Gruppe der Reste folgender Duftstoffalkohole: 10-Undecen-1-ol, 2,6-Dimethylheptan-2-ol, 2-Methyl-butanol, 2-Methylpentanol, 2-Phenoxyethanol, 2-Phenylpropanol, 2-tert-Butycyclohexanol, 3,5,5-Trimethylcyclohexanol, 3-Hexanol, 3-Methyl-5-phenyl-pentanol, 3-Octanol, 3-Phenyl-propanol, 4-Heptenol, 4-Isopropylcyclohexanol, 4-tert.-Butycyclohexanol, 6,8-Dimethyl-2-nona-nol, 6-Nonen-1-ol, 9-Decen-1-ol, α-Methylbenzylalkohol, α-Terpineol, Amylsalicylat, Benzylalkohol, Benzylsalicylat, β-Terpineol, Butylsalicylat, Citronellol, Cyclohexylsalicylat, Decanol, Dihydromyrcenol, Dimethylbenzylcarbinol, Dimethylheptanol, Dimethyloctanol, Ethylsalicylat, Ethylvanillin, Eugenol, Farnesol, Geraniol, Heptanol, Hexylsalicylat, Isoborneol, Isoeugenol, Isopulegol, Linalool, Menthol, Myrtenol, n-Hexanol, Nerol, Nonanol, Octanol, p-Menthan-7-ol, Phenylethylalkohol, Phenol, Phenylsalicylat, Tetrahydrogeraniol, Tetrahydrolinalool, Thymol, trans-2-cis-6-Nonadicnol, trans-2-Nonen-1-ol, trans-2-Octenol, Undecanol, Vanillin, Champiniol, Hexenol und/oder Zimtalkohol.

6. Mischungen aus einer Kombination aus Verbindungen der allgemeinen Formel (I) und (II) gemäß einem der Ansprüche 1 bis 5 und Verbindungen der allgemeinen Formel (III) 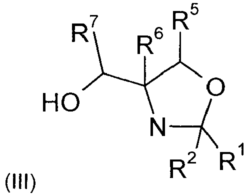 mit den in der allgemeinen Formel (I) angegebenen Bedeutungen für R¹, R², R⁵, R⁶, R⁷.

7. Verwendung von Kombinationen nach einem der Ansprüche 1 bis 5 oder Mischungen nach Anspruch 6 als Duftstoffvorläufer.

8. Verwendung nach Anspruch 7, **dadurch gekennzeichnet, dass** die Kombination Duftstoff-Aldehyde und Duftstoff-Alkohole als Duftstoffe freisetzen.

9. Verwendung nach Anspruch 7, **dadurch gekennzeichnet, dass** die Kombination Duftstoff-Ketone und Duftstoff-Alkohole als Duftstoffe freisetzen.

10. Verwendung nach einem der vorhergehenden Ansprüche 8 bis 10, **dadurch gekennzeichnet, dass** die Kombinationen zusammen mit anderen Duftstoffen eingesetzt werden.

11. Verwendung nach einem der vorhergehenden Ansprüche 8 bis 11 in Wasch- oder Reinigungsmitteln, Weichspülern und Kosmetika.

12. Wasch- oder Reinigungsmittel, Weichspüler oder Kosmetika, enthaltend Kombinationen nach einem der Ansprüche 1 bis 5 oder Mischungen nach Anspruch 6.

13. Wasch- oder Reinigungsmittel, Weichspüler oder Kosmetika nach Anspruch 12, **dadurch gekennzeichnet, dass** die Kombinationen der Verbindung der allgemeinen Formel (I) und (II) oder Mischungen derer mit Verbindungen der allgemeinen Formel (III) in Mengen von weniger als 5 Gew.-%, vorzugsweise weniger als 2 Gew.-%, insbesondere weniger als 1 Gew.-%, jeweils bezogen auf die Gesamtmenge der Mittel, in den Mittel enthalten ist.

14. Verfahren zur Verlängerung des Duftempfindens von Wasch- oder Reinigungsmitteln, Weichspülern oder Kosmetika oder mit diesen behandelten festen Oberflächen, **dadurch gekennzeichnet, dass** den Wasch- oder Reinigungsmitteln, Weichspülern oder Kosmetika Kombinationen nach einem der Ansprüche 1 bis 5 oder Mischungen nach Anspruch 6 zugesetzt werden.

15. Verfahren zum Abbau von Schlechtgerüchen durch Einsatz einer Kombination aus
(a) 1-Aza-3,7-dioxabicyclo[3.3.0]octan-Verbindung der allgemeinen Formel (I) 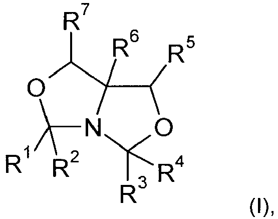 wobei
R¹, R², R³, R⁴ unabhängig voneinander für Reste stehen, die in einer Verbindung der allgemeinen Formel R¹-C(=O)-R² oder R³-C(=O)-R⁴ einen Duftstoff-Aldehyd mit mindestens 6 Kohlenstoffatomen oder ein Duftstoff-Keton mit mindestens 6 Kohlenstoffatomen ergeben, R⁵, R⁶, R⁷ unabhängig voneinander für H oder einen Kohlenwasserstoff-Rest stehen, der acyclisch oder cyclisch, substituiert oder unsubstituiert, verzweigt oder unverzweigt sowie gesättigt oder ungesättigt sein kann
oder Gemische dieser Verbindungen und
(b) einem Kieselsäureester der allgemeinen Formel (II) 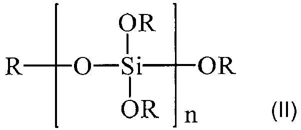 wobei
n Werte im Bereich von 2 bis 100 annimmt und
alle R unabhängig voneinander ausgewählt sind aus der Gruppe aus Wasserstoff, geradkettigen oder verzweigten, gesättigten oder ungesättigten, substituierten oder unsubstituierten C₁₋₆-Kohlenwasserstoffresten und Duftstoffalkoholresten, unter der Maßgabe, dass mindestens ein R für einen Duftstoffalkoholrest steht,
oder Gemische dieser Verbindungen.

## Claims

1. A combination of
(a) a 1-aza-3,7-dioxabicyclo[3.3.0]octane compound of the general formula (I) 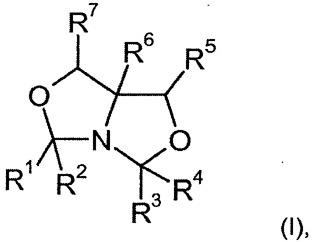 wherein
R¹, R², R³, R⁴ mutually independently denote groups that, in a compound of the general formula R¹-C(=O)-R² or R³-C(=O)-R⁴, yield a scent aldehyde having at least six carbon atoms or a scent ketone having at least six carbon atoms,
R⁵, R⁶, R⁷ mutually independently denote hydrogen or a hydrocarbon group that can be acyclic or cyclic, substituted or unsubstituted, branched or unbranched as well as saturated or unsaturated,
or mixtures of said compounds, and
(b) a silicic acid ester of the general formula (II) 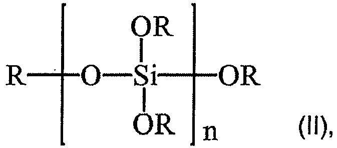 wherein
n assumes values in the range from 2 to 100, and
all R are selected mutually independently from the group of hydrogen, straight-chain or branched, saturated or unsaturated, substituted or unsubstituted C₁₋₆ hydrocarbon groups and scent alcohol groups, with the provision that at least one R denotes a scent alcohol group,
or mixtures of said compounds.

2. The combination according to Claim 1, wherein R¹, R², R³, R⁴ mutually independently denote groups that, in a compound of the general formula R¹-C(=O)-R² or R³-C(=O)-R⁴, respectively yield a scent aldehyde having at least 6 carbon atoms.

3. The combination according to Claim 1, **characterized in that** groups R¹ and R² or R³ and R⁴ that yield a scent ketone in a compound of the general formula R¹-C(=O)-R² or R³-C(=O)-R⁴, respectively, are present at most in one of the structural elements -CR¹R² or -CR³R⁴, respectively.

4. The combination according to one of Claims 1 to 3, **characterized in that** in the compound of the general formula (I) the groups R¹, R², R³ and R⁴ yield, in a compound of the general formula R¹-C(=O)-R² or R³-C(=O)-R⁴, a scent aldehyde or a scent ketone that is selected from the list made up of jasmonenes; ionones, damascones and damascenones, menthone, carvone, Iso-E-Super, methylheptenone, melonal, cymal, ethyl vanillin, helional, hydroxycitronellal, koavone, methylnonylacetaldehyde, phenylacetaldehyde, undecylenealdehyde, 3-dodecene-1-al, alpha-n-amylcinnamaldehyde, benzaldehyde, 3-(4-tert-butylphenyl)propanal, 2-methyl-3-(para-methoxyphenylpropanal), 2-methyl-4-(2,6,6-trimethyl-2(1)-cyclohexen-1-yl)butanal, 3-phenyl-2-propenal, cis-/trans-3,7-dimethyl-2,6-octadien-1-al, 3,7-dimethyl-6-octen-1-al, [(3,7-dimethyl-6-octenyl)oxy]acetaldehyde, 4-isopropylbenzaldehyde, 2,4-dimethyl-3-cyclohexene-1-carboxaldehyde, 2-methyl-3-(isopropylphenyl)propanal, decylaldehyde, 2,6-dimethyl-5-heptenal, alpha-n-hexylcinnamaldehyde, 7-hydroxy-3,7-dimethyloctanal, undecenal, 2,4,6-trimethyl-3-cyclohexene-1-carboxaldehyde, 1-dodecanal, 2,4-dimethylcyclohexene-3-carboxaldehyde, 4-(4-hydroxy-4-methylpentyl)-3-cylohexene-1-carboxaldehyde, 2-methylundecanal, 2-methyldecanal, 1-nonanal, 1-octanal, 2,6,10-trimethyl-5,9-undecadienal, 2-methyl-3-(4-tertbutyl)propanal, dihydrocinnamaldehyde, 3,7-dimethyloctan-1-al, 1-undecanal, 10-undecen-1-al, 4-hydroxy-3-methoxybenzaldehyde, trans-4-decenal, 2,6-nonadienal, para-tolylacetaldehyde, 3,7-dimethyl-2-methylene-6-octenal, 2-methyloctanal, alpha-methyl-4-(1-methylethyl)benzeneacetaldehyde, 2-methyl-3-phenyl-2-propen-1-al, 3,5,5-trimethylhexanal, 3-propylbicyclo[2.2.1]hept-5-ene-2-carbaldehyde, 9-decenal, 3-methyl-5-phenyl-1-pentanal, methylnonylacetaldehyde, citral, 1-decanal, florhydral, 2,4-dimethyl-3-cyclohexene-1-carboxaldehyde, heliotropin.

5. The combination according to one of Claims 1 to 4, **characterized in that** in the silicic acid ester of the general formula (II) at least 10 mol%, preferably at least 20 mol% and particularly preferably even more than 40 mol% of the groups R are selected from the group of the groups of the following scent alcohols: 10-undecen-1-ol, 2,6-dimethylheptan-2-ol, 2-methylbutanol, 2-methylpentanol, 2-phenoxyethanol, 2-phenylpropanol, 2-tert-butycyclohexanol, 3,5,5-trimethylcyclohexanol, 3-hexanol, 3-methyl-5-phenylpentanol, 3-octanol, 3-phenylpropanol, 4-heptenol, 4-isopropylcyclohexanol, 4-tert-butylcyclohexanol, 6,8-dimethyl-2-nonanol, 6-nonen-1-ol, 9-decen-1-ol, α-methylbenzyl alcohol, α-terpineol, amyl salicylate, benzyl alcohol, benzyl salicylate, β-terpineol, butyl salicylate, citronellol, cyclohexyl salicylate, decanol, dihydromyrcenol, dimethylbenzylcarbinol, dimethylheptanol, dimethyloctanol, ethyl salicylate, ethyl vanillin, eugenol, farnesol, geraniol, heptanol, hexyl salicylate, isoborneol, isoeugenol, isopulegol, linalool, menthol, myrtenol, n-hexanol, nerol, nonanol, octanol, p-menthan-7-ol, phenylethyl alcohol, phenol, phenyl salicylate, tetrahydrogeraniol, tetrahydrolinalool, thymol, trans-2-cis-6-nonadienol, trans-2-nonen-1-ol, trans-2-octenol, undecanol, vanillin, champiniol, hexenol, and/or cinnamyl alcohol.

6. Mixtures of a combination of compounds of the general formulas (I) and (II) according to one of Claims 1 to 5 and compounds of the general formula (III) 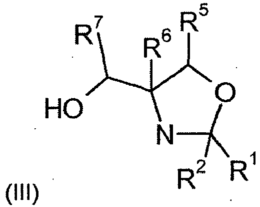 having the meanings for R¹, R², R⁵, R⁶, R⁷ indicated in the general formula (I).

7. Use of combinations according to one of Claims 1 to 5, or of mixtures according to Claim 6, as a scent precursor.

8. The use according to Claim 7, **characterized in that** that the combination releases scent aldehydes and scent alcohols as scents.

9. The use according to Claim 7, **characterized in that** the combination releases scent ketones and scent alcohols as scents.

10. The use according to one of the preceding claims 8 to 9, **characterized in that** the combinations are employed together with other scents.

11. The use according to one of the preceding Claims 8 to 11 in washing or cleaning agents, fabric softeners, and cosmetics.

12. Washing or cleaning agents, fabric softeners, or cosmetics containing combinations according to one of Claims 1 to 5 or mixtures according to Claim 6.

13. Washing or cleaning agents, fabric softeners, or cosmetics according to Claim 12, **characterized in that** the combinations of the compound of the general formula (I) and (II), or mixtures thereof with compounds of the general formula (III), are contained in the agent in quantities of less than 5 wt%, preferably less than 2 wt%, in particular less than 1 wt%, based in each case on the total quantity of the agents.

14. A method for extending the scent sensation of washing or cleaning agents, fabric softeners, or cosmetics, or of solid surfaces treated therewith, **characterized in that** combinations according to one of Claims 1 to 5 or mixtures according to Claim 6 are added to the washing or cleaning agents, fabric softeners, or cosmetics.

15. A method for degrading bad odors by using a combination of
(a) a 1-aza-3,7-dioxabicyclo[3.3.0]octane compound of the general formula (I) 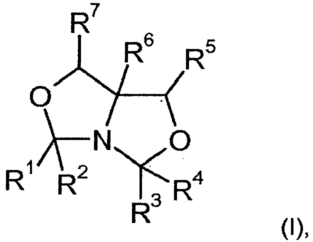 wherein
R¹, R², R³, R⁴ mutually independently denote groups that, in a compound of the general formula R¹-C(=O)-R² or R³-C(=O)-R⁴, yield a scent aldehyde having at least six carbon atoms or a scent ketone having at least six carbon atoms,
R⁵, R⁶, R⁷ mutually independently denote hydrogen or a hydrocarbon group that can be acyclic or cyclic, substituted or unsubstituted, branched or unbranched as well as saturated or unsaturated,
or mixtures of said compounds, and
(b) a silicic acid ester of the general formula (II) 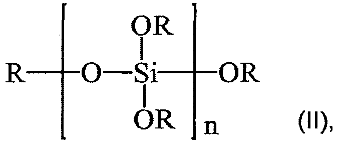 wherein
n assumes values in the range from 2 to 100, and
all R are selected mutually independently from the group of hydrogen, straight-chain or branched, saturated or unsaturated, substituted or unsubstituted C₁₋₆ hydrocarbon groups and scent alcohol groups, with the provision that at least one R denotes a scent alcohol group,
or mixtures of said compounds.

## Revendications

1. Combinaison
(a) d'un composé 1-aza-3,7-dioxabicyclo[3.3.0]octane de la Formule générale (I) 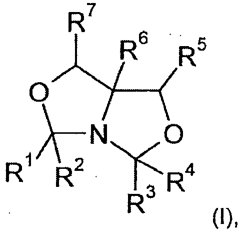 dans laquelle
R¹, R², R³, R⁴ représentent indépendamment les uns des autres des résidus qui, dans un composé de la Formule générale R¹-C(=O)-R² ou R³-C(=O)-R⁴, résultent en un aldéhyde parfumé avec au moins 6 atomes de carbone ou une cétone parfumée avec au moins 6 atomes de carbone,
R⁵, R⁶, R⁷ représentent indépendamment les uns des autres H ou un résidu hydrocarbure qui peut être acyclique ou cyclique, substitué ou non substitué, ramifié ou non ramifié ainsi que saturé ou insaturé
ou de mélanges de ces composés et
(b) d'un ester d'acide silicique de la Formule générale (II) 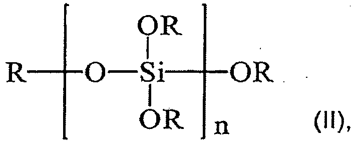 dans laquelle
n prend des valeurs comprises dans la gamme allant de 2 à 100 et
tous les R sont sélectionnés indépendamment les uns des autres dans le groupe de l'hydrogène, de résidus alcool parfumé et de résidus hydrocarbure en C₁ à C₆ à chaîne droite ou ramifiés, saturés ou insaturés, substitués ou non substitués, à la condition qu'au moins un R représente un résidu alcool parfumé,
ou de mélanges de ces composés.

2. Combinaison selon la revendication 1, dans laquelle R¹, R², R³, R⁴ représentent indépendamment les uns des autres des résidus qui, dans un composé de la Formule générale R¹-C(=O)-R² ou R³-C(=O)-R⁴, résultent à chaque fois en un aldéhyde parfumé avec au moins 6 atomes de carbone.

3. Combinaison selon la revendication 1, **caractérisée en ce que** les résidus R¹ et R², respectivement R³ et R⁴, sont présents au maximum dans l'un des éléments structuraux -CR¹R², respectivement -CR³R⁴, qui, dans un composé de la Formule générale R¹-C(=O)-R², respectivement R³-C(=O)-R⁴, résultent en une cétone parfumée.

4. Combinaison selon l'une des revendications 1 à 3, **caractérisée en ce que** les résidus R¹, R², R³ et R⁴ dans le composé de la Formule générale (I) résultent, dans un composé de la Formule générale R¹-C(=O)-R² ou R³-C(=O)-R⁴, en un aldéhyde parfumé ou une cétone parfumée, lequel ou laquelle est sélectionné(e) dans la liste constituée par les jasmones ; les ionones, les damascones et les damascénones, la menthone, la carvone, l'Iso-E-Super, la méthylhepténone, le mélonal, le cymal, l'éthylvanilline, l'hélional, l'hydroxycitronellal, la koavone, le méthylnonylacétaldéhyde, le phénylacétaldéhyde, l'undécylénaldéhyde, le 3-dodécén-1-al, l'alpha-n-amylcinnamaldéhyde, le benzaldéhyde, le 3-(4-tert-butylphényl)-propanal, le 2-méthyl-3-(para-méthoxyphénylpropanal), le 2-méthyl-4-(2,6,6-triméthyl-2(1)-cyclohexén-1-yl)butanal, le 3-phényl-2-propénal, le cis-/trans-3,7-diméthyl-2,6-octadién-1-al, le 3,7-diméthyl-6-octén-1-al, le [(3,7-diméthyl-6-octényl)oxy]acétaldéhyde, le 4-isopropylbenzaldéhyde, le 2,4-diméthyl-3-cyclohexène-1-carboxaldéhyde, le 2-méthyl-3-(isopropylphényl)propanal, le décylaldéhyde, le 2,6-diméthyl-5-hepténal, l'alpha-n-hexylcinnamaldéhyde, le 7-hydroxy-3,7-diméthyloctanal, l'undécénal, le 2,4,6-triméthyl-3-cyclohexène-1-carboxaldéhyde, le 1-dodécanal, le 2,4-diméthylcyclohexène-3-carboxaldéhyde, le 4-(4-hydroxy-4-méthylpentyl)-3-cyclohexène-1-carboxaldéhyde, le 2-méthylundécanal, le 2-méthyldécanal, le 1-nonanal, le 1-octanal, le 2,6,10-triméthyl-5,9-undécadiénal, le 2-méthyl-3-(4-tert-butyl)propanal, le dihydrocinnamaldéhyde, le 3,7-diméthyloctan-1-al, le 1-undécanal, le 10-undécén-1-al, le 4-hydroxy-3-méthoxybenzaldéhyde, le trans-4-décénal, le 2,6-nonadiénal, le para-tolylacétaldéhyde, le 3,7-diméthyl-2-méthylène-6-octénal, le 2-méthyloctanal, l'alpha-méthyl-4-(1-méthyléthyl)-benzène acétaldéhyde, le 2-méthyl-3-phényl-2-propén-1-al, le 3,5,5-triméthylhexanal, le 3-propyl-bicyclo[2.2.1]hept-5-ène-2-carbaldéhyde, le 9-décénal, le 3-méthyl-5-phényl-1-pentanal, le méthylnonylacétaldéhyde, le citral, le 1-décanal, le florhydral, le 2,4-diméthyl-3-cyclohexène-1-carboxaldéhyde, l'héliotropine.

5. Combinaison selon l'une des revendications 1 à 4, **caractérisée en ce que**, dans l'ester d'acide silicique de la Formule générale (II), au moins 10 % en moles, de préférence au moins 20 % en moles et de manière particulièrement préférée même plus de 40 % en moles des résidus R sont sélectionnés dans le groupe des résidus d'alcools parfumés suivants: le 10-undécén-1-ol, le 2,6-diméthylheptan-2-ol, le 2-méthylbutanol, le 2-méthylpentanol, le 2-phénoxyéthanol, le 2-phénylpropanol, le 2-tert-butylcyclohexanol, le 3,5,5-triméthylcyclohexanol, le 3-hexanol, le 3-méthyl-5-phénylpentanol, le 3-octanol, le 3-phénylpropanol, le 4-hepténol, le 4-isopropylcyclohexanol, le 4-tert-butylcyclohexanol, le 6,8-diméthyl-2-nonanol, le 6-nonén-1-ol, le 9-décén-1-ol, l'alcool α-méthylbenzylique, l'α-terpinéol, le salicylate d'amyle, l'alcool benzylique, le salicylate de benzyle, le β-terpinéol, le salicylate de butyle, le citronellol, le salicylate de cyclohexyle, le décanol, le dihydromyrcénol, le diméthylbenzylcarbinol, le diméthylheptanol, le diméthyloctanol, le salicylate d'éthyle, l'éthylvanilline, l'eugénol, le farnésol, le géraniol, l'heptanol, le salicylate d'hexyle, l'isobornéol, l'isoeugénol, l'isopulégol, le linalol, le menthol, le myrténol, le n-hexanol, le nérol, le nonanol, l'octanol, le p-menthan-7-ol, l'alcool phényléthylique, le phénol, le salicylate de phényle, le tétrahydrogéraniol, le tétrahydrolinalol, le thymol, le trans-2-cis-6-nonadiénol, le trans-2-nonén-1-ol, le trans-2-octénol, l'undécanol, la vanilline, le champiniol, l'hexénol et/ou l'alcool cinnamique.

6. Mélanges d'une combinaison de composés des Formules générales (I) et (II) selon l'une des revendications 1 à 5 et de composés de la Formule générale (III) 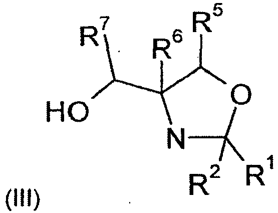 avec les significations données dans la Formule générale (I) pour R¹, R², R⁵, R⁶, R⁷.

7. Utilisation de combinaisons selon l'une des revendications 1 à 5 ou de mélanges selon la revendication 6 en tant que précurseur de substance parfumée.

8. Utilisation selon la revendication 7, **caractérisée en ce que** la combinaison libère des aldéhydes parfumés et des alcools parfumés en tant que substances parfumées.

9. Utilisation selon la revendication 7, **caractérisée en ce que** la combinaison libère des cétones parfumées et des alcools parfumés en tant que substances parfumées.

10. Utilisation selon l'une des revendications précédentes 8 à 10, **caractérisée en ce que** les combinaisons sont utilisées conjointement avec d'autres substances parfumées.

11. Utilisation selon l'une des revendications précédentes 8 à 11 dans des détergents ou des produits de nettoyage, des assouplissants et des produits cosmétiques.

12. Détergent ou produit de nettoyage, assouplissant ou produit cosmétique, contenant des combinaisons selon l'une des revendications 1 à 5 ou des mélanges selon la revendication 6.

13. Détergent ou produit de nettoyage, assouplissant ou produit cosmétique selon la revendication 12, **caractérisé en ce que** les combinaisons du composé des Formules générales (I) et (II) ou des mélanges de celles-ci avec des composés de la Formule générale (III) sont contenus dans les produits dans des quantités de moins de 5 % en poids, de préférence de moins de 2 % en poids, en particulier de moins de 1 % en poids, rapporté respectivement à la quantité totale des produits.

14. Procédé pour le prolongement de la sensation parfumée de détergents ou de produits de nettoyage, d'assouplissants ou de produits cosmétiques ou de surfaces solides traitées avec ceux-ci, **caractérisé en ce que** des combinaisons selon l'une des revendications 1 à 5 ou des mélanges selon la revendication 6 sont ajoutés aux détergents ou produits de nettoyage, assouplissants ou produits cosmétiques.

15. Procédé pour la réduction des mauvaises odeurs grâce à l'utilisation d'une combinaison
(a) d'un composé 1-aza-3,7-dioxabicyclo[3.3.0]octane de la Formule générale (I) 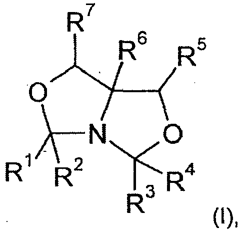 dans laquelle
R¹, R², R³, R⁴ représentent indépendamment les uns des autres des résidus qui, dans un composé de la Formule générale R¹-C(=O)-R² ou R³-C(=O)-R⁴, résultent en un aldéhyde parfumé avec au moins 6 atomes de carbone ou une cétone parfumée avec au moins 6 atomes de carbone,
R⁵, R⁶, R⁷ représentent indépendamment les uns des autres H ou un résidu hydrocarbure qui peut être acyclique ou cyclique, substitué ou non substitué, ramifié ou non ramifié ainsi que saturé ou insaturé ou de mélanges de ces composés et
(b) d'un ester d'acide silicique de la Formule générale (II) 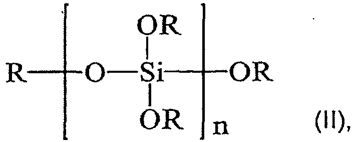 dans laquelle
n prend des valeurs comprises dans la gamme allant de 2 à 100 et
tous les R sont sélectionnés indépendamment les uns des autres dans le groupe de l'hydrogène, de résidus alcool parfumé et de résidus hydrocarbure en C₁ à C₆ à chaîne droite ou ramifiés, saturés ou insaturés, substitués ou non substitués, à la condition qu'au moins un R représente un résidu alcool parfumé,
ou de mélanges de ces composés.
